# EUROPEAN PATENT SPECIFICATION

(11) **EP 3 804 731 B1**
(45) Date of publication and mention of the grant of the patent: **04.11.2026**
(21) Application number: 19814441.2
(22) Date of filing: 16.05.2019
(51) Int. Cl.: A61K 33/00, A61K 9/00, A61K 47/14, A61K 47/32, A61K 47/38, A61P 17/02

(54) **COMPOSITION COMPRISING CARBON MONOXIDE FOR USE IN TREATING SKIN WOUNDS**
ZUSAMMENSETZUNG UMFASSEND KOHLENMONOXID ZUR VERWENDUNG BEI DER BEHANDLUNG VON HAUTWUNDEN
COMPOSITION COMPRENANT DU MONOXYDE DE CARBONE POUR SON UTILISATION DANS LE TRAITEMENT DE PLAIES CUTANÉES

(30) Priority: 08.06.2018 JP 2018110717
(43) Date of publication of application: 14.04.2021
(73) Proprietor: Sumitomo Seika Chemicals Co., Ltd., Kako-gun, Hyogo 675-0145 (JP); Kyoto Prefectural Public University Corporation, Kyoto-shi, Kyoto 602-8566 (JP)
(72) Inventor: TAKAGI, Tomohisa, Kyoto-shi, Kyoto 602-8566 (JP); ASAI, Jun, Kyoto-shi, Kyoto 602-8566 (JP); OKAYAMA, Tetsuya, Kyoto-shi, Kyoto 602-8566 (JP); MIURA, Katsura, Kyoto-shi, Kyoto 602-8566 (JP); SAKAUE, Shigeki, Kako-gun, Hyogo 675-0145 (JP)
(74) Representative: Vossius & Partner Patentanwälte Rechtsanwälte mbB
(86) International application number: PCT/JP2019/019579
(87) International publication number: WO 2019/235166

(56) References cited:
- EP-A1- 1 043 023
- WO-A1-03/094932
- JP-A- 2014 508 135
- JP-A- 2017 516 747
- JASINSKI KRYSTIAN ET AL: "Impact of gaseous nitric oxide and carbon monoxide on normal excisional, diabetic excisional and burn wound healing", 1 November 2009 (2009-11-01), XP093195319, Retrieved from the Internet <URL:chrome-extension://efaidnbmnnnibpcajpcglclefindmkaj/https://etd.ohiolink.edu/acprod/odb_etd/ws/send_file/send?accession=ohiou1256055541&disposition=inline> [retrieved on 20240902]
- AHANGER AA ET AL.: "Wound healing activity of carbon monoxide liberated from CO-releasing molecule (CO-RM", NAUNYN-SCHMIEDEBERG'S ARCH PHARMACOL, vol. 384, 2011, pages 93 - 102, XP019916687, DOI: 10.1007/s00210-011-0653-7

## Description

### Technical Field

The present invention relates to a composition for external use in preventing and/or treating skin wounds, the composition comprising carbon monoxide and a solvent, wherein the carbon monoxide concentration is 1000 µM or more.

### Background Art

A wound or ulcer that is damage to skin tissue etc. is usually called a "wound." Specific examples include erosion, cuts, abrasions, burns, skin ulcers, pressure ulcers, and the like.

In particular, skin ulcers and pressure ulcers are caused by being bedridden or having diabetes, arteriosclerosis obliterans, vasculitis, and the like. Since many of these conditions are prolonged, the number of patients will surely increase, which is a major social problem, with the advent of the super aging society.

However, skin ulcers and pressure ulcers are still intractable diseases.

In the current situation, a sufficient therapeutic effect on intractable skin ulcers, pressure ulcers, etc. often cannot be expected with conservative treatment using a preparation for external use for treating wounds, such as an agent combining sucrose and povidone-iodine (Patent Literature (PTL) 1). When the condition becomes severe, surgical treatment is required (e.g., skin transplantation). Therefore, the development of a novel preparation for external use for skin that exhibits a more excellent therapeutic effect has been in demand. PTL 2 relates to the use of carbon monoxide as a biomarker and therapeutic agent of heart, lung, liver, spleen, brain, skin and kidney diseases.

### Citation List

### Patent Literature

PTL 1: JP2000-38342A
PTL 2: WO 03/094932 A1

### Non-patent Literature

NPL 1: Journal of Investigative Dermatology, (2006) 126, 1159-1167

### Summary of Invention

### Technical Problem

The present inventors found that a CO aqueous solution obtained by dissolving a high concentration of CO in water is effective for external use in preventing and/or treating inflammatory gastrointestinal diseases. Therefore, the inventors attempted to prevent and/or treat skin ulcers by applying the same aqueous solution containing a high concentration of CO to skin ulcers.

An object of the present invention is to provide a novel means capable of treating skin ulcers. Thus, the present invention relates to a composition for external use in preventing and/or treating skin wounds, the composition comprising carbon monoxide and a solvent, wherein the carbon monoxide concentration is 1000 µM or more.

### Solution to Problem

The present inventors found the possibility that a CO-containing composition may be capable of treating skin ulcers. The inventors made further improvements and completed the present invention, which is as defined in the claims.

### Advantageous Effects of Invention

The composition for external use in preventing and/or treating skin wounds encompassed by the present invention exhibits an excellent preventive and/or therapeutic effect.

### Brief Description of Drawings

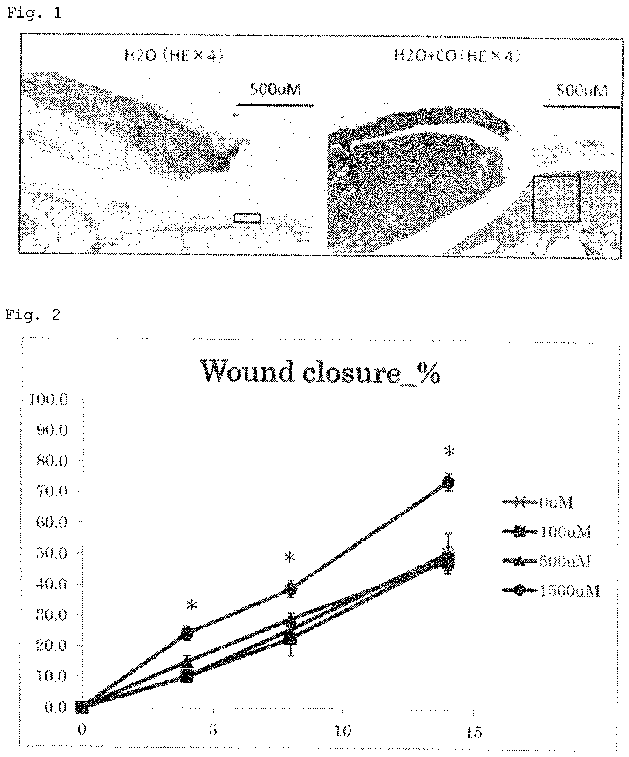

Fig. 1 shows the results of hematoxylin eosin (HE) staining showing recovery from ulcers when a CO-containing composition (solvent: water) was applied to the ulcer site of skin ulcer model mice.
Fig. 2 is a graph showing the percentage (%) of an ulcer recovery (wound closure) area when a CO-containing composition (solvent: a carbomer aqueous solution) was applied to the ulcer site of skin ulcer model mice. The horizontal axis shows the number of days, and the vertical axis shows the percentage (%) of the ulcer recovery area with respect to the initial ulcer area.

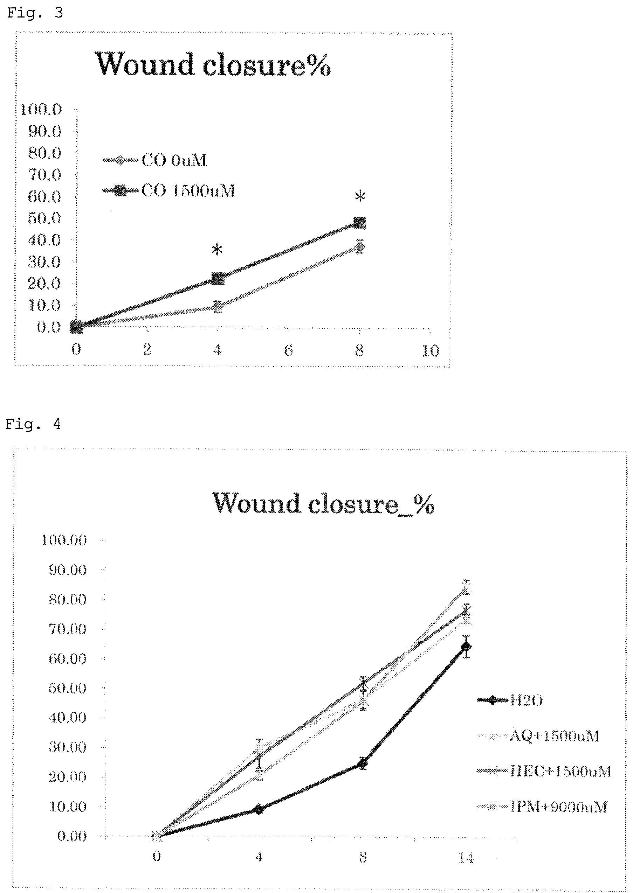

Fig. 3 is a graph showing the percentage (%) of an ulcer recovery (wound closure) area when a CO-containing composition (solvent: a hydroxyethyl cellulose aqueous solution) was applied to the ulcer site of skin ulcer model mice. The horizontal axis shows the number of days, and the vertical axis shows the percentage (%) of the ulcer recovery area with respect to the initial ulcer area.
Fig. 4 is a graph showing the percentage (%) of an ulcer recovery (wound closure) area when a CO-containing composition (solvent: a carbomer aqueous solution, a hydroxyethyl cellulose aqueous solution, or isopropyl myristate) was applied to the ulcer site of skin ulcer model mice. The horizontal axis shows the number of days, and the vertical axis shows the percentage (%) of the ulcer recovery area with respect to the initial ulcer area.

### Description of Embodiments

Below, each embodiment of the present invention is described in more detail.

The composition for external use in preventing and/or treating skin wounds (such as skin ulcers) encompassed by the present invention comprises carbon monoxide (CO) and a solvent. This composition may be referred to as "the composition of the present invention".

The solvent contained in the composition of the present invention is not particularly limited as long as it can retain (preferably dissolve) CO. Examples include water, organic solvents, and liquid mixtures of these. For liquid mixtures, the combination of solvents is not particularly limited. Further, the liquid mixtures can be homogeneous or heterogeneous. Examples of organic solvents include saturated aliphatic hydrocarbons, fatty acids, vegetable oils, carboxylic acid esters, alcohols, ethers, ketones, glycols, fatty acid esters, and the like. Examples of fatty acid esters include esters of fatty acids containing 12 to 18 carbon atoms and alkyl alcohols containing 1 to 20 carbon atoms. The alkyl alcohol particularly preferably contains 1 to 6 carbon atoms from the viewpoint of economy, safety, etc. The fatty acids may be saturated fatty acids or unsaturated fatty acids (preferably containing 1, 2, or 3 unsaturated bonds). More specific examples include lauric acid, myristic acid, palmitic acid, stearic acid, oleic acid, and the like. The alkyl alcohol may be straight-chain or branched-chain alkyl alcohol. More specific examples include methanol, ethanol, n-propanol, isopropanol, n-butanol, isobutanol, n-pentanol, isopentanol, n-hexanol, isohexanol, and the like. Particularly preferable examples of fatty acid esters include isopropyl myristate, isopropyl palmitate, and the like. Specific examples of organic solvents other than fatty acid esters include diisopropyl adipate, acetone, benzyl benzoate, isopropanol, fennel oil, almond oil, ethanol, ethylene glycol, 2-ethyl-1,3-hexanediol, diethyl ether, octyldodecanol, olive oil, oleic acid, glycerin fatty acid ester, crotamiton, geraniol-modified alcohol, synthetic squalane, sesame oil, wheat germ oil, ethyl acetate, normal butyl acetate, safflower oil, safflower oil fatty acid, ethylene glycol salicylate, diisopropanolamine, diethylene glycol, diethylene glycol monobutyl ether, diethylene glycol monomethyl ether, cyclohexanone, perilla oil, dipropylene glycol, dimethylpolysiloxane, squalene, diethyl sebacate, sorbitan acid fatty acid ester, soybean oil, soybean lecithin, propylene carbonate, medium-chain fatty acid triglyceride, camellia oil, corn oil, dodecylbenzene, triacetin, sorbitan trioleate, glyceryl tricaprylate, trichloroethane, concentrated glycerin, peppermint oil, octaacetyl sucrose-modified alcohol, castor oil, 1,3-butylene glycol, propionic acid, propylene glycol, propylene glycol fatty acid ester, benzyl alcohol, polyoxyethylene oleyl ether, polyoxyethylene sorbitan monostearate, polysorbate, polyethylene glycol, ethanol, methanol, methyl isobutyl ketone, methyl ethyl ketone, cottonseed oil, α-monoisostearyl glyceryl ether, polyethylene glycol monooleate, sorbitan monolaurate, coconut oil, polyoxyethylene lauryl alcohol ether, peanut oil, liquid paraffin, and the like. In particular, the solvent is preferably water or isopropyl myristate from the viewpoint of economy and ability to retain CO.

The composition of the present invention may further comprise a thickener. Examples of thickeners include monosaccharides and derivatives thereof, polysaccharides and derivatives thereof, fatty acids, amino acids and derivatives thereof, sugar alcohols, fatty acid esters, saturated aliphatic hydrocarbons, aliphatic alcohols, inorganic compounds, and the like. Specific examples include xanthan gum, gum arabic, guar gum, carrageenan, gellan gum, agar, locust bean gum, carbomer (carboxyvinyl polymer), carboxymethyl cellulose, sodium carboxyethyl cellulose, shellac, methyl cellulose, hydroxyethyl cellulose, hydroxypropyl cellulose, hydroxypropylmethyl cellulose, polyvinylpyrrolidone, polyvinyl alcohol, acrylic acid-alkyl methacrylate copolymer, polyacrylic acid salt, sodium alginate, propylene glycol alginate, ethyl cellulose, sodium carboxymethyl cellulose, magnesium aluminum silicate, sodium chondroitin sulfate, α-cyclodextrin, concentrated glycerin, polyethylene glycol, liquid paraffin, carob bean gum, glucono-δ-lactone, light anhydrous silicic acid, squalane, stearyl alcohol, aluminum stearate, lanolin, cetyl alcohol, gelatin, dextrin, urea, Vaseline, palmitic acid, potato starch, sodium hyaluronate, hydroxyethyl methylcellulose, castor oil, 1,3-butylene glycol, propylene glycol, polysorbate, polyethylene glycol, sodium metaphosphate, starch syrup, beeswax, methyl vinyl ether-maleic anhydride copolymer, rosin, dextrin palmitate, dextrin myristate, inulin stearate, 12-hydroxystearic acid, 1,3:2,4-dibenzylidene-D-sorbitol, L-leucine derivatives, L-isoleucine derivatives, L-valine derivatives, N-lauroyl-L-glutamic acid-α, and the like. Carbomers and hydroxyethyl cellulose are particularly preferable from the viewpoint of economy, safety, etc. These thickeners may be used alone, or in a combination of two or more.

For example, when water is used as the solvent, and a carbomer is used as the thickener, the composition preferably comprises 0.05 to 30 mass% of carbomer.

Further, for example, when water is used as the solvent, and hydroxyethyl cellulose is used as the thickener, the composition preferably comprises 0.1 to 14 mass% of hydroxyethyl cellulose.

The composition of the present invention preferably has a viscosity of about 0.3 to 1000000 centipoise (cp), more preferably about 0.5 to 500000 cp, and even more preferably about 0.8 to 100000 cp. The viscosity is a value measured by using a Brookfield viscometer after allowing the composition to be measured to stand at 25°C for 30 minutes.

The composition of the present invention that comprises water as a solvent and that further comprises a thickener preferably has a viscosity within the above range although there is no particular limitation to this. The type and amount of the thickener used are also not particularly limited. It is preferable to appropriately select and use a thickener so that the viscosity falls within this range of the viscosity.

The composition of the present invention is preferably a composition for external use. Further, the composition of the present invention is preferably a liquid composition. The liquid composition as used herein includes a viscous composition (viscous liquid composition).

The composition of the present invention comprises CO in a concentration of 1000 µM or more. The CO concentration may be, for example, 1050 µM or more, 1100 µM or more, 1150 µM or more, 1200 µM or more, 1250 µM or more, 1300 µM or more, 1350 µM or more, 1400 µM or more, 1450 µM or more, or 1500 µM or more. The upper limit of the CO concentration is not particularly limited, and CO can be incorporated into the solvent until saturation is achieved. The upper limit is, for example, 10000 µM or less, 9500 µM or less, or 9000 µM or less.

To obtain such a composition with a high CO concentration, for example, simply bringing CO into contact with, or blowing CO into, a solvent under ordinary pressure is usually insufficient. To prepare such a composition with a high CO concentration, it is preferable, for example, to perform replacement of CO by adjusting the pressure to, for example, 2 atm (or more) by using a gas-tight syringe or the like in a sealed container in which CO is collected.

The composition of the present invention is preferably for external use in preventing and/or treating skin ulcers. The cause of the skin ulcers is not particularly limited. Examples of the causes include external injury, burns, diabetes, radiation exposure, peripheral vascular lesions, such as arteriosclerosis and venous stasis, collagen diseases (e.g., rheumatism), and the like. The covering epithelial damage that is milder than an ulcer is usually called "erosion" when the damage remains microscopically in the epithelial mucosa and does not reach the lower layer although the epithelium is macroscopically deficient. In the present specification, the term "ulcer" is used in the sense of including erosion unless otherwise specified.

The composition of the present invention can be applied not only to humans but also to non-human mammals. Examples of the non-human mammals include mice, rats, cats, dogs, monkeys, cows, horses, and the like.

The composition of the present invention can be administered, for example, once or multiple times (for example, 2 or 3 times) per day. The dose can be set appropriately. For example, the amount of the composition is preferably about 0.1 to 10 cc/day. As described above, the composition of the present invention is for external use. The administration is performed by, for example, applying the composition to an ulcer site. The composition of the present invention may also be, for example, a liquid formulation or a foam-like formulation (foam formulation). The enema-foam-like formulation can be applied to the skin by ejecting a mixture of a liquid composition and an injection gas from a spray nozzle. For ejection, in particular, it is often the case that the liquid composition and the injection gas are mixed in a spray bottle immediately before ejection. The preferable CO concentration of the composition of the present invention in the form of a foam formulation may be the CO concentration obtained after mixing the liquid composition and the injection gas. Specifically, the CO concentration is 1000 µM or more after the liquid composition and the injection gas are mixed. Further, CO may be contained in the liquid composition or the injection gas, or both.

The composition of the present invention may further comprise other components as long as the effect of the present invention is not impaired. For other such components, known components can be used. In particular, components known to be used in compositions for external use can be used. Examples include disintegrating agents, lubricants (anti-aggregation agents), fluidizing agents, pH-adjusting agents, tonicity agents, absorption promoters, coloring agents, flavoring agents, antioxidants, antibacterial agents, preservatives, and the like.

Examples of disintegrating agents include carmellose calcium, low-substituted hydroxypropylcellulose, carmellose, croscarmellose sodium, partially pregelatinized starch, dried starch, sodium carboxymethyl starch, crospovidone, polysorbate 80 (polyoxyethylene sorbitan oleate), and the like.

Examples of lubricants (anti-aggregation agents) include talc, magnesium stearate, calcium stearate, colloidal silica, stearic acid, hydrous silicon dioxide, synthetic magnesium silicate, fine-particle silicon oxide, starch, sodium lauryl sulfate, boric acid, magnesium oxide, waxes, hydrogenated oil, polyethylene glycol, sodium benzoate, and the like.

Examples of fluidizing agents include silicic acid anhydride and the like.

Examples of pH-adjusting agents include hydrochloric acid, sodium hydroxide, citric acid, anhydrous citric acid, sodium citrate, sodium citrate dihydrate, anhydrous sodium monohydrogen phosphate, anhydrous sodium dihydrogen phosphate, and the like.

Examples of tonicity agents include sodium chloride, glucose, D-mannitol, glycerin, and the like.

Examples of absorption promoters include quaternary ammonium base, sodium lauryl sulfate, and the like. The content of these other components in the composition can be appropriately determined according to their type.

In the present specification, the term "comprise" or "contain" includes the meanings of consisting essentially of and consisting of.

### Examples

The present invention is described in more detail below.

### 1. Preparation of CO-containing Composition

### Method for preparing H₂O + CO sample

Pure water (2.5 mL) was placed in a vial (3 mL, produced by Maruemu Corporation), and the vial was sealed with a rubber stopper and an aluminum seal. Next, a gas (N₂ balance) having an arbitrarily adjusted CO concentration was collected in a Tedlar bag (1 L, produced by AS ONE Corporation). The air in the gas phase in the vial was replaced with the gas in the Tedlar bag until pressure reached about 2 atm by using a gas-tight syringe (MS-GAN500, produced by Ito Corporation). Subsequently, the resulting product was allowed to stand until CO in the gas phase was dissolved in the liquid phase to reach equilibrium.

Table 1 shows the CO concentrations of the obtained CO-containing compositions (solvent: water). The CO concentration of the compositions was analyzed by gas chromatography (GC-2014 FID produced by Shimadzu Corporation). These examples are according to the invention in as far as the carbon monoxide concentration is 1000 µM or more.

**Table 1**

| CO in gas phase in Tedlar bag (%) (theoretical value) | | 8 | 34 | 67 | 100 |
|---|---|---|---|---|---|
| CO concentration of liquid phase obtained by actual measurement (µM) | Solvent (H₂O) | 100 | 500 | 1000 | 1500 |

### Method for preparing IPM + CO sample

Isopropyl myristate (IPM: Wako first grade, produced by Fujifilm Wako Pure Chemical Corporation) (2.5 mL) was placed in a vial (3 mL, produced by Maruemu Corporation), and the vial was sealed with a rubber stopper and an aluminum seal. Next, a gas (N₂ balance) having an arbitrarily adjusted CO concentration was collected in a Tedlar bag (1 L, produced by AS ONE Corporation). The air in the gas phase in the vial was replaced with the gas in the Tedlar bag until pressure reached about 2 atm by using a gas-tight syringe (MS-GAN500, produced by Ito Corporation). Subsequently, the resulting product was allowed to stand until CO in the gas phase was dissolved in the liquid phase to reach equilibrium.

The CO concentration of the obtained compositions was analyzed by gas chromatography (GC-2014 FID produced by Shimadzu Corporation). When the CO (%) in the gas phase in the Tedlar bag was 18%, the CO concentration of IPM was 1500 µM. When the CO (%) in the gas phase in the Tedlar bag was 100%, the CO concentration of IPM was 9000 µM.

### Administration to Skin Ulcer Model Mice - Example 1

Following a known method (Asai et al., Journal of Investigative Dermatology (2006) 126, 1159-1167), skin ulcer model mice were prepared. Specifically, spontaneously diabetic male mice (db/db mice) were received at 7 weeks of age, their backs were shaved, and the mice were then preliminarily kept in individual cages until 8 weeks of age. Each mouse was intraperitoneally injected with a mixed solution of ketamine and ceracetal, and the back of the mouse was shaved again under anesthesia. Then, a biopsy punch was used to induce a full-thickness skin defect wound with a diameter of 8 mm, thus producing skin ulcer model mice. Below, the thus-produced skin ulcer model mice were used as model mice.

The CO-containing composition (solvent: water, containing CO in a concentration of 1500 µM) was applied to the ulcer site of the skin ulcer model mice. The ulcer site was covered with a film (hydrophilic polyurethane dressing material; IV3000 dressing, transparent dressing) (without suturing). Thereafter, an operation of washing the ulcer site with physiological saline and applying the composition was repeated every other day. As a control experiment, an experiment was conducted in the same manner as above, except that water was used instead of the CO-containing composition.

On day 14 from the application of the CO-containing composition, hematoxylin eosin (HE) staining of the ulcer sites was performed. Fig. 1 shows the results. The group to which the CO-containing composition was applied showed promoted formation of granulation tissue, compared to the group to which only water was applied.

### 2. Preparation of CO-containing Composition

### Method for Preparing Carbomer + CO Sample

Pure water (300 g) was placed in a 500-mL poly beaker. While stirring was performed at 400 rpm with a three-one motor (BL-1200, produced by Shinto Scientific Co., Ltd.), 2.79 g of carbomer (Aqupec, 501E, produced by Sumitomo Seika Co., Ltd.) was added and dissolved by stirring at 240 rpm for 5 hours. Thereafter, 15.8 g of 6% sodium hydroxide aqueous solution was added and stirred for 30 minutes to prepare a carbomer aqueous solution. The carbomer aqueous solution (150 mL) was placed in a 200-mL beaker, and the resulting product was allowed to stand in a constant temperature water tank at 25°C for 30 minutes.

The carbomer aqueous solution (2.5 mL) was placed in a vial (3 mL, produced by Maruemu Corporation), and the vial was sealed with a rubber stopper and an aluminum seal. Next, a gas (N₂ balance) having an arbitrarily adjusted CO concentration was collected in a Tedlar bag (1 L, produced by AS ONE Corporation). The air in the gas phase in the vial was replaced with the gas in the Tedlar bag until pressure reached about 2 atm by using a gas-tight syringe (MS-GAN500, produced by Ito Corporation). Subsequently, the resulting product was allowed to stand until CO in the gas phase was dissolved in the liquid phase to reach equilibrium.

Table 2 shows the CO concentrations of the obtained CO-containing compositions. The CO concentration of the compositions was analyzed by gas chromatography (GC-2014 FID produced by Shimadzu Corporation).

**Table 2**

| CO in gas phase in Tedlar bag (%) (theoretical value) | | 8 | 35 | 100 |
|---|---|---|---|---|
| CO concentration in liquid phase obtained by actual measurement (µM) | Solvent (carbomer aqueous solution) | 100 | 500 | 1500 |

### Method for Preparing Hydroxyethyl Cellulose + CO Sample

Pure water (250 g) was placed in a 500-mL poly beaker. While stirring was performed at 400 rpm with a three-one motor (BL-1200, produced by Shinto Scientific Co., Ltd.), 5.10 g of hydroxyethyl cellulose (CF-W, produced by Sumitomo Seika Chemicals Company, Limited) was added and dissolved by stirring at 240 rpm for 10 hours to prepare a hydroxyethyl cellulose aqueous solution. The hydroxyethyl cellulose aqueous solution (150 mL) was placed in a 200-mL beaker, and the resulting product was allowed to stand in a constant temperature water tank at 25°C for 30 minutes.

The hydroxyethyl cellulose aqueous solution (2.5 mL) was placed in a vial (3 mL, produced by Maruemu Corporation), and the vial was sealed with a rubber stopper and an aluminum seal. Next, a gas (N₂ balance) having an arbitrarily adjusted CO concentration was collected in a Tedlar bag (1 L, produced by AS ONE Corporation). The air in the gas phase in the vial was replaced with the gas in the Tedlar bag until pressure reached about 2 atm by using a gas-tight syringe (MS-GAN500, produced by Ito Corporation). Subsequently, the resulting product was allowed to stand until CO in the gas phase was dissolved in the liquid phase to reach equilibrium.

The CO concentration of the obtained CO-containing compositions was analyzed by gas chromatography (GC-2014 FID produced by Shimadzu Corporation). When the CO (%) in the gas phase in the Tedlar bag was 100%, the CO concentration in the hydroxyethyl cellulose aqueous solution was 1500 µM.

### Administration to Skin Ulcer Model Mice - Example 2

The CO-containing composition that comprised carbomer prepared as described above was applied to the ulcer site of the skin ulcer model mice in the same manner as in Example 1. On days 4, 8, and 14, the area of the ulcer site (wound closure) was measured. As a control experiment, an experiment was conducted in the same manner as above, except that a composition that comprised only water and carbomer (the carbomer aqueous solution) was used instead of the CO-containing composition. Fig. 2 shows the results. The use of the composition that comprised only water and carbomer or the CO-containing composition that had a CO concentration of 100 µM showed almost no therapeutic effect on the skin ulcer. However, the use of the CO-containing composition that had a CO concentration of 500 µM showed a difference in the degree of recovery from the initial wound. Furthermore, the use of the CO-containing composition that had a CO concentration of 1500 µM showed a significant therapeutic effect on the skin ulcer. In Fig. 2, the asterisk (*) indicates a significant difference with respect to the group to which the composition comprising only water and carbomer was applied (P<0.05). These examples are according to the invention in as far as the carbon monoxide concentration is 1000 µM or more.

### Administration to Skin Ulcer Model Mice - Example 3

Further, analysis was performed using the skin ulcer model mice in the same manner as in Example 2, except that the CO-containing composition that comprised hydroxyethyl cellulose prepared as described above (CO concentration: 1500 µM) was used. Fig. 3 shows the results. The use of the CO-containing composition that had a CO concentration of 1500 µM showed a significant therapeutic effect on the skin ulcer. In Fig. 3, the asterisk (*) indicates a significant difference with respect to the group to which the composition that comprised only water and hydroxyethyl cellulose (the hydroxyethyl cellulose aqueous solution) was applied (P<0.05).

### Administration to Skin Ulcer Model Mice - Example 4

Analysis was performed using the skin ulcer model mice in the same manner as in Example 2, except that the CO-containing composition that comprised carbomer prepared as described above (solvent: water, CO concentration: 1500 µM), the CO-containing composition that comprised hydroxyethyl cellulose prepared as described above (solvent: water, CO concentration: 1500 µM), the CO-containing composition that comprised isopropyl myristate as the solvent (CO concentration: 9000 µM), or water was used. Fig. 4 shows the results. All of the CO-containing compositions showed a significant therapeutic effect on skin ulcer, compared to water. In Fig. 4, the CO-containing composition that comprised carbomer (solvent: water, CO concentration: 1500 µM), the CO-containing composition that comprised hydroxyethyl cellulose (solvent: water, CO concentration: 1500 µM), and the CO-containing composition that comprised isopropyl myristate as the solvent (CO concentration: 9000 µM) were denoted as "AQ + 1500 µM," "HEC + 1500 µM," and "IPM + 9000 µM," respectively.

## Claims

1. A composition for external use in preventing and/or treating skin wounds, the composition comprising carbon monoxide and a solvent, wherein the carbon monoxide concentration is 1000 µM or more.

2. The composition for external use in preventing and/or treating skin wounds according to claim 1, wherein the solvent is water.

3. The composition for external use in preventing and/or treating skin wounds according to claim 1, wherein the solvent is isopropyl myristate.

4. The composition for external use in preventing and/or treating skin wounds according to any one of claims 1 to 3, further comprising a thickener.

5. The composition for external use in preventing and/or treating skin wounds according to claim 4, wherein the thickener is at least one member selected from the group consisting of carboxyvinyl polymers and hydroxyalkyl cellulose.

6. The composition for external use in preventing and/or treating skin wounds according to any one of claims 1 to 5, wherein the carbon monoxide concentration is 1500 µM or more.

7. The composition for external use in preventing and/or treating skin wounds according to any one of claim 1 to 6, wherein the composition is to be applied to the skin wound.

8. The composition for external use in preventing and/or treating skin wounds according to any one of claims 1 to 7, wherein the skin wound is a skin ulcer.

9. The composition for external use in preventing and/or treating skin wounds according to claim 8, wherein the composition is to be applied to an ulcer site.

## Patentansprüche

1. Eine Zusammensetzung zur äußerlichen Verwendung bei der Vorbeugung und/oder Behandlung von Hautwunden, wobei die Zusammensetzung Kohlenmonoxid und ein Lösungsmittel umfasst, wobei die Kohlenmonoxidkonzentration 1000 µM oder mehr beträgt.

2. Die Zusammensetzung zur äußerlichen Verwendung bei der Vorbeugung und/oder Behandlung von Hautwunden gemäß Anspruch 1, wobei das Lösungsmittel Wasser ist.

3. Die Zusammensetzung zur äußerlichen Verwendung zur Vorbeugung und/oder Behandlung von Hautwunden gemäß Anspruch 1, wobei das Lösungsmittel Isopropylmyristat ist.

4. Die Zusammensetzung zur äußerlichen Verwendung bei der Vorbeugung und/oder Behandlung von Hautwunden gemäß einem der Ansprüche 1 bis 3, die ferner ein Verdickungsmittel umfasst.

5. Die Zusammensetzung zur äußerlichen Verwendung bei der Vorbeugung und/oder Behandlung von Hautwunden gemäß Anspruch 4, wobei das Verdickungsmittel mindestens ein Mitglied ist, ausgewählt aus der Gruppe, bestehend aus Carboxyvinylpolymeren und Hydroxyalkylcellulose.

6. Die Zusammensetzung zur äußerlichen Verwendung bei der Vorbeugung und/oder Behandlung von Hautwunden gemäß einem der Ansprüche 1 bis 5, wobei die Kohlenmonoxidkonzentration 1500 µM oder mehr beträgt.

7. Die Zusammensetzung zur äußerlichen Verwendung bei der Vorbeugung und/oder Behandlung von Hautwunden gemäß einem der Ansprüche 1 bis 6, wobei die Zusammensetzung auf die Hautwunde aufzutragen ist.

8. Die Zusammensetzung zur äußerlichen Verwendung bei der Vorbeugung und/oder Behandlung von Hautwunden gemäß einem der Ansprüche 1 bis 7, wobei die Hautwunde ein Hautgeschwür ist.

9. Die Zusammensetzung zur äußerlichen Verwendung bei der Vorbeugung und/oder Behandlung von Hautwunden gemäß Anspruch 8, wobei die Zusammensetzung auf eine Geschwürstelle aufzutragen ist.

## Revendications

1. Composition pour son utilisation externe dans la prévention et/ou le traitement des plaies cutanées, la composition comprenant du monoxyde de carbone et un solvant, dans laquelle la concentration en monoxyde de carbone est de 1000 µM ou plus.

2. Composition pour son utilisation externe dans la prévention et/ou le traitement des plaies cutanées selon la revendication 1, dans laquelle le solvant est de l'eau.

3. Composition pour son utilisation externe dans la prévention et/ou le traitement des plaies cutanées selon la revendication 1, dans laquelle le solvant est du myristate d'isopropyle.

4. Composition pour son utilisation externe dans la prévention et/ou le traitement des plaies cutanées selon l'une quelconque des revendications 1 à 3, comprenant en outre un épaississant.

5. Composition pour son utilisation externe dans la prévention et/ou le traitement des plaies cutanées selon la revendication 4, dans laquelle l'épaississant est au moins un élément choisi dans le groupe constitué par des polymères carboxyvinyliques et d'hydroxyalkylcellulose.

6. Composition pour son utilisation externe dans la prévention et/ou le traitement des plaies cutanées selon l'une quelconque des revendications 1 à 5, dans laquelle la concentration en monoxyde de carbone est de 1500 µM ou plus.

7. Composition pour son utilisation externe dans la prévention et/ou le traitement des plaies cutanées selon l'une quelconque des revendications 1 à 6, dans laquelle la composition doit être appliquée à la plaie cutanée.

8. Composition pour son utilisation externe dans la prévention et/ou le traitement des plaies cutanées selon l'une quelconque des revendications 1 à 7, dans laquelle la plaie cutanée est un ulcère cutané.

9. Composition pour son utilisation externe dans la prévention et/ou le traitement des plaies cutanées selon la revendication 8, dans laquelle la composition doit être appliquée à un site ulcéreux.
